# DEMANDE DE BREVET EUROPEEN

(11) **EP 1 001 266 A1**
(43) Date de publication de la demande: **17.05.2000**
(21) Numéro de dépôt: 99402634.2
(22) Date de dépôt: 22.10.1999
(51) Int. Cl.: G01N 33/24

(54) **Dispositif pour tester des échantillons de roches à pression et température relativement élevées**

(30) Priorité: 09.11.1998 FR 9814114
(71) Demandeur: INSTITUT FRANCAIS DU PETROLE, 92852 Rueil-Malmaison Cedex (FR)
(72) Inventeur: Fernandes, Gérard, 92000 Nanterre (FR); Brandely, José, 91600 Savigny sur Orge (FR); Garnier, Dominique, 78630 Orgeval (FR)

(57) **Abrégé**

- Dispositif pour l'étude de propriétés physiques d'un échantillon solide (S) en présence de fluides, à température et pression relativement élevées.
- Le dispositif comporte un corps rigide allongé (1) fermé à ses extrémités opposées par deux couvercles (2, 3), une gaine souple (9) autour de l'échantillon, coopérant avec deux pièces d'embout (7, 8) pour délimiter à l'intérieur du corps rigide, une enceinte de confinement, des moyens d'injection pour déplacer des fluides sous pression au travers de l'échantillon, dans sa gaine, des moyens de mesure pour mesurer différents paramètres de l'échantillon et des moyens (P) d'injection d'un fluide sous pression autour de la gaine souple (9) de façon à exercer une pression radiale et éventuellement axiale autour de l'échantillon, des moyens de chauffage du fluide sous pression associés à au moins un des deux couvercles (3, 4) et des moyens incluant une turbine (12) par exemple, pour homogénéiser la température autour de la gaine par circulation du fluide chauffé au contact des moyens de chauffage. Le corps est réalisé de préférence en matériau composite.
- Applications à des études d'échantillons géologiques par exemple.

## Description

La présente invention concerne un dispositif pour l'étude de propriétés physiques d'échantillons solides en présence de fluides, à température et pression élevées.

Le dispositif selon l'invention convient pour tester par exemple des échantillons géologiques et déterminer différents paramètres tels que la pression capillaire des roches dans des phases de drainage ou d'imbibition, leurs indices de mouillabilité, leurs perméabilités relatives, leurs indices de résistivité etc, dans des conditions de température et de pression reproduisant celles que l'on trouve dans des formations recelant ou susceptibles de receler des hydrocarbures où ils ont été prélevés.

Pour déterminer la répartition des volumes d'huile et de gaz dans un gisement, il faut connaître en tout point les valeurs des saturations qui dépendent de paramètres tels que la mouillabilité et la tension interfaciale. A cet effet, on détermine la mouillabilité des roches vis à vis de l'eau et de l'huile qui peuvent y être contenues. Il faut procéder pour cela à des opérations de drainage de la roche c'est-à-dire à un déplacement des fluides visant à diminuer la saturation en eau, suivi d'opérations d'imbibition, en désignant par ce terme un déplacement des fluides permettant d'augmenter la saturation en eau (Sw) de la roche. La pression capillaire en un point d'un matériau poreux contenant deux fluides tels que de l'eau et de l'huile en phase continue, se définit, on le rappelle, comme la différence Pc à l'équilibre entre la pression P(huile) de l'huile et celle P(eau) de l'eau.

La connaissance de différents paramètres et notamment de la mouillabilité des roches, est utile notamment quand on doit procéder à une récupération assistée d'une formation, en drainant les effluents qu'elle contient par une injection d'un fluide sous pression, et que l'on doit par des tests préalables choisir le fluide (eau ou gaz) qui convient le mieux pour déplacer les effluents.

Pour mener à bien ces opérations de drainage et d'imbibition, en laboratoire, différents types de dispositifs sont utilisés. Les barreaux d'échantillons sont placés généralement dans une cellule. A ses extrémités opposées, la cellule comporte deux embouts communiquant avec des moyens pour établir un déplacement de fluides sous pression au travers de l'échantillon testé. Des moyens de mesure sont utilisés pour mesurer différents paramètres: pressions, saturations, résistivité électrique etc. Le barreau d'échantillon peut être placé à l'intérieur d'une gaine de confinement en élastomère comprimée par une injection de fluide sous pression.

Différentes dispositifs de mesure de paramètres physiques d'échantillons solides poreux sont décrits par exemple dans les brevet FR.2 708 742, FR 2 724 460 (US 5 610 524) ou FR 2 728 684 (US 5 637 796) du demandeur.

Le suivi du déplacement des fluides à l'intérieur d'un échantillon est souvent réalisé en utilisant des moyens d'investigation par rayonnement X ou gamma extérieurs à la cellule. Les parois du corps de cellule doivent être suffisamment résistantes pour supporter des pressions de confinement de plusieurs dizaines de Mpa tout en restant transparentes au rayonnement et donc relativement minces.

Les moyens classiques pour maintenir l'échantillon à une température relativement élevée (150°C par exemple): étuve ou manchons chauffants enroulés autour du corps, sont difficilement compatibles avec l'utilisation d'un appareil d'investigation à rayonnement du fait qu'ils se trouvent interposés entre lui et l'échantillon et contribuent encore plus à l'atténuation des rayons

Le dispositif selon l'invention permet de réaliser des mesures des propriétés physiques d'un échantillon à pression et températures relativement élevées, en évitant notamment les inconvénients des dispositifs antérieurs.

Le dispositif comporte un corps rigide allongé fermé à ses extrémités opposés par deux pièces d'embout, une gaine souple autour de l'échantillon, coopérant avec les deux pièces d'embout pour délimiter à l'intérieur du corps rigide, une enceinte de confinement, des moyens pour établir un déplacement de fluides sous pression au travers de l'échantillon dans son enceinte, des moyens de mesure pour mesurer différents paramètres de l'échantillon et des moyens d'injection d'un fluide sous pression autour de la gaine souple de façon à exercer une pression radiale autour de l'échantillon.

Le dispositif est caractérisé en ce qu'il comporte des moyens de chauffage du fluide sous pression (tels que des résistances électriques) associés à au moins un des deux couvercles et des moyens pour homogénéiser la température autour de la gaine par circulation du fluide chauffé au contact des moyens de chauffage.

Les moyens de circulation de fluide comportent par exemple un moyen de brassage coopérant avec un circuit destiné à faire circuler le fluide au contact des moyens de chauffage.

Suivant un mode de réalisation, le moyen de brassage comporte une turbine actionnée par un moteur, qui est disposée dans une cavité de l'un des deux couvercles, et le circuit destiné à faire circuler le fluide au contact des moyens de chauffage comporte un tube disposé dans l'espace annulaire entre le corps et la gaine, ce tube faisant communiquer des conduits intérieurs aux deux couvercles.

Suivant un mode préféré de réalisation, le corps comporte une partie tubulaire réalisée en un matériau composite, les deux couvercles étant réalisés en un matériau bon conducteur de la chaleur.

Les couvercles peuvent être réunis au corps soit directement soit par des tirants qui peuvent être aussi en matériau composite.

Le dispositif comporte par exemple des canaux au travers des deux couvercles pour la mise en communication des extrémités de l'échantillon avec des moyens de circulation de fluide.

Les moyens de mesure peuvent comporter un appareil de mesure de l'absorption d'un rayonnement par le dit échantillon.

Le mode de chauffage utilisé est avantageux car il est simple et efficace. Les moyens de chauffage sont au contact direct de l'échantillon ce qui permet de diminuer le temps nécessaire pour obtenir une répartition de température bien uniforme. En outre, aucun élément extérieur ne se trouve interposé, susceptible de venir absorber des rayonnements émis par des appareils de mesure extérieurs au corps, la sensibilité des mesures faites par rayonnement est améliorée.

L'utilisation d'un corps réalisé en un matériau composite permet aussi de limiter l'épaisseur requise compatible avec les pressions élevées nécessaires à la conduite des opérations de mesure. La faible absorption d'un tel matériau aux émis par les appareils de mesure permet en outre de faciliter leur mise en oeuvre.

D'autres caractéristiques et avantages du dispositif selon l'invention, apparaîtront à la lecture de la description ci-après d'un exemple non limitatif de réalisation, en se référant aux dessins annexés où:

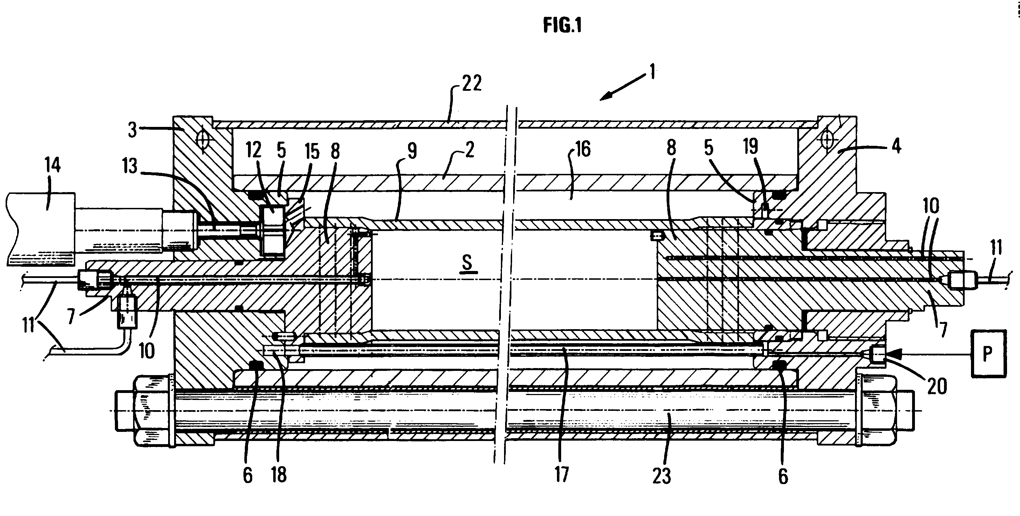

- la Fig.1 montre une vue en coupe longitudinale de la cellule ; et
- la Fig.2 montre une vue en coupe transversale d'un couvercle d'extrémité avec des moyens de chauffage intégrés.

Le dispositif comporte un corps creux 1 constitué d'une partie tubulaire 2 fermée à ses deux extrémités opposées par deux couvercles 3, 4 pourvus chacun d'un épaulement 5 muni de joints d'étanchéité 6, venant s'emboîter étroitement à l'intérieur de la partie tubulaire 2. Le dispositif comporte deux pièces d'embout réalisées en un matériau bon conducteur de la chaleur, constituée chacune d'une tige 7 et d'une tête 8 de section plus grande que celle de la tige mais inférieure à celle de la partie tubulaire 2. Chaque couvercle 3, 4 comporte un alésage axial pour une tige 7. L'échantillon S à tester est placé dans une gaine souple 9 en élastomère par exemple, dont les extrémités opposées sont enfilées sur les deux têtes 8. Chaque pièce d'embout est traversée de part en part par des canaux 10 et de moyens de connexion pour conduits extérieurs 11 reliés à des moyens de pompage (non représentés), permettent de déplacer des fluides à l'intérieur de l'échantillon S, comme décrit dans les brevets antérieurs précédemment cités.

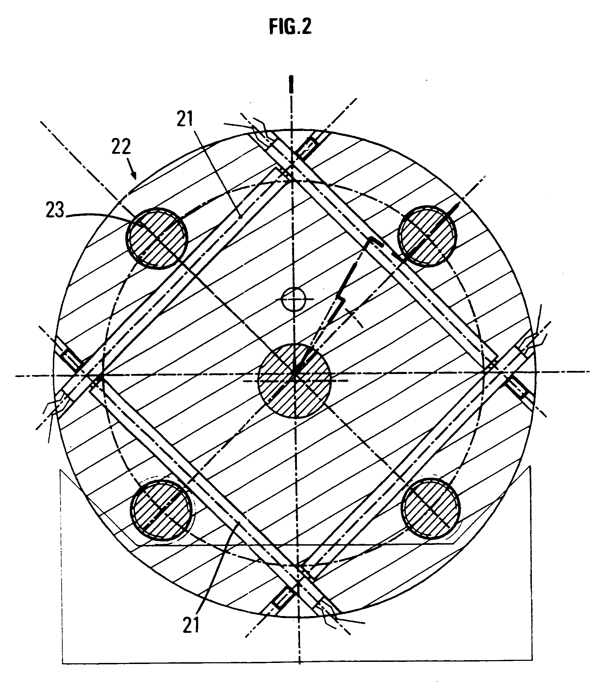

Une cavité est ménagée dans le couvercle 3 pour une turbine 12. Un alésage 13 parallèle à l'axe du corps 1, est ménagé au travers du couvercle 3, permettant la connexion de l'axe de la turbine avec un moteur d'entraînement 14. La cavité de la turbine communique par un canal 15 avec l'espace annulaire 16 entre la gaine 9 et la partie tubulaire 2. Un tube fin 17 est disposé dans l'espace annulaire 16 entre les têtes 8 des deux pièce d'embout. Il communique à une première extrémité avec la cavité de la turbine 12 par l'intermédiaire d'une rainure circulaire 18 dans le couvercle 3, et à son extrémité opposée, avec l'espace annulaire 16 par l'intermédiaire d'une deuxième rainure circulaire 19 dans le deuxième couvercle 4. Un canal 20 au travers du deuxième couvercle 4, permet de mettre en communication l'espace annulaire 16 avec des moyens d'injection P d'un fluide sous pression (de l'huile par exemple) destinée à exercer une pression radiale sur l'échantillon S par l'intermédiaire de la gaine souple 9. Des passages (Fig.2) sont ménagés dans chacun des couvercles 3, 4 pour des résistances électriques 21 connectées à une alimentation électrique (non représentée).

Les deux couvercles d'extrémité 3, 4 sont emboîtés dans un cylindre 22 formant entretoise et maintenus fermement contre la partie tubulaire 2, par plusieurs tirants 23.

Le dispositif fonctionne de la façon suivante:

L'échantillon S ayant été positionné entre les deux têtes des pièces d'embout et les couvercles ayant été ajustés et fixés au corps, on injecte par le canal 20 du fluide sous pression dans l'espace annulaire 16. On alimente alors les résistances électriques 21 servant à chauffer les deux couvercles 3, 4 et on actionne la turbine 12. Le fluide circule d'une extrémité à l'autre de l'espace annulaire 16 par l'intermédiaire du tube 17 et des voies de communication d'extrémité 15, 18, 19. Il est réchauffé en permanence à son passage par l'intérieur des deux couvercles 3, 4 qui sont portées à température élevée par les résistances électriques 23. Par ce brassage et ce chauffage permanents, l'huile de l'espace annulaire 16 et par conséquent l'échantillon S peuvent être portés rapidement à une température de consigne bien homogène reproduisant par exemple celle qui règne dans le sous-sol à la profondeur où il a été prélevé.

Un appareil d'analyse de l'échantillon par rayonnement peut être placé à la périphérie du cylindre 22 dans les secteurs angulaires entre les tirants 23

La partie tubulaire 2 ainsi que le cylindre 22 sont réalisés de préférence dans un matériau composite. On bénéficie de sa rigidité intrinsèque pour diminuer leur épaisseur et du même coup leur capacité d'absorption des ondes émises par les appareils d'examen par rayonnement que l'on peut disposer autour du dispositif. On bénéficie aussi d'une conductibilité thermique moindre que celle des métaux utilisés (l'aluminium par exemple), ce qui a pour effet bénéfique, de diminuer les pertes calorifiques par rayonnement et donc d'atteindre plus vite la température de consigne nécessaire pour les besoins des essais.

Les tirants 23 peuvent aussi être réalisés dans un matériau composite de manière à ne plus faire obstacle à la pénétration du rayonnement des appareils de mesure et autoriser ainsi des déplacements sur toute la périphérie du corps rigide.

On a décrit un agencement où des tirants 23 sont utilisés pour solidariser les couvercles et la partie périphérique 2 du corps 1. On peut préférer cependant un mode de fixation direct des couvercles au corps, permettant de libérer tout à fait la périphérie du corps, ce qui permet d'obtenir un dispositif plus compact et de simplifier la mise en place d'appareils d'examen de l'échantillon autour et le long de l'échantillon S.

## Revendications

1. Dispositif pour l'étude de propriétés physiques d'un échantillon solide (S) en présence de fluides, à température et pression relativement élevées, comportant un corps rigide allongé (1) fermé à ses extrémités opposés par deux couvercles (3, 4), une gaine souple (9) autour de l'échantillon, coopérant avec deux pièces d'embout (7, 8) pour délimiter à l'intérieur du corps rigide, une enceinte de confinement, des moyens pour établir un déplacement de fluides sous pression au travers de l'échantillon dans sa gaine, des moyens de mesure pour mesurer différents paramètres de l'échantillon et des moyens (P) d'injection d'un fluide sous pression dans un espace annulaire autour de la gaine souple (9) de façon à exercer une pression radiale autour de l'échantillon, caractérisé en ce qu'il comporte des moyens de chauffage du fluide sous pression associés à au moins un des deux couvercles (3, 4) et des moyens de circulation pour faire circuler autour de la gaine le fluide sous pression chauffé au contact des moyens de chauffage et homogénéiser la température de l'échantillon.

2. Dispositif selon la revendication 1, caractérisé en ce que les moyens de chauffage comportent des résistances électriques associées à un moins un des couvercles (3, 4).

3. Dispositif selon l'une des revendications précédentes, caractérisé en ce que les moyens de circulation de fluide comportent un moyen de brassage (12) coopérant avec un circuit (15, 17-19) destiné à faire circuler le fluide au contact des moyens de chauffage.

4. Dispositif selon la revendication 3, caractérisé en ce que le moyen de brassage (12) comporte une turbine actionnée par un moteur (14), qui est disposée dans une cavité de l'un des deux couvercles (3), et le circuit destiné à faire circuler le fluide au contact des moyens de chauffage comporte un tube (17) disposé dans l'espace annulaire entre le corps (1) et la gaine (9), ce tube (17) faisant communiquer entre eux des conduits (15, 18, 19) intérieurs aux deux couvercles (3, 4).

5. Dispositif selon l'une des revendications précédentes, caractérisé en ce que le corps (1) comporte une partie tubulaire (2) réalisée en un matériau composite, les deux couvercles (3, 4)étant réalisés en un matériau bon conducteur de la chaleur.

6. Dispositif selon l'une des revendications précédentes, caractérisé en ce que les couvercles sont fixés au corps (1) par des tirants (23).

7. Dispositif selon la revendication précédente, caractérisé en ce que les tirants (23) sont réalisés dans un matériau composite.

8. Dispositif selon la revendication 5, caractérisé en ce qu'il comporte des moyens de fixation directs des deux couvercles (3, 4) à la partie tubulaire (2) du corps.

9. Dispositif selon l'une des revendications précédentes, caractérisé en ce qu'il comporte des canaux (10) au travers des deux couvercles (3, 4) pour la mise en communication des extrémités de l'échantillon (S) avec des moyens de circulation de fluide.

10. Dispositif selon l'une des revendications précédentes, caractérisé en ce que les moyens de mesure comportent un appareil de mesure de l'absorption d'un rayonnement par le dit échantillon (S).
